(19) Europäisches Patentamt
European Patent Office
Office européen des brevets

(11) Veröffentlichungsnummer: **0 363 793 B1**

(12) # EUROPÄISCHE PATENTSCHRIFT

(45) Veröffentlichungstag der Patentschrift: **21.04.93**

(51) Int. Cl.$^5$: **C07D 311/16**, A61K 31/37

(21) Anmeldenummer: **89118359.2**

(22) Anmeldetag: **04.10.89**

(54) **Arylalkoxycumarine, Verfahren zu ihrer Herstellung und diese enthaltende therapeutische Mittel.**

(30) Priorität: **13.10.88 DE 3834861**

(43) Veröffentlichungstag der Anmeldung:
**18.04.90 Patentblatt 90/16**

(45) Bekanntmachung des Hinweises auf die Patenterteilung:
**21.04.93 Patentblatt 93/16**

(84) Benannte Vertragsstaaten:
**AT BE CH DE FR GB IT LI NL**

(56) Entgegenhaltungen:
**DE-A- 2 805 485**
**US-A- 4 200 577**

**CHEMICAL ABSTRACTS, Band 100, Nr. 13, 26. MUrz 1984, Columbus, Ohio, USA MATSUSHITA ELECTRIC INDU- STRIAL CO. "Coumarinyl ester derivatives" Seite 635, Spalte 1, Zusammenfassung-Nr. 103 179c**

**CHEMICAL ABSTRACTS, Band 100, Nr. 11, 12. MUrz 1984, Columbus, Ohio, USA DELALANDE S.A. "Phenyl amino- alkyl ethers and their therapeutic use" Seite 516, Spalte 2, Zu- sammenfassung-Nr. 85 390h**

(73) Patentinhaber: **BASF Aktiengesellschaft**
**Carl-Bosch-Strasse 38**
**W-6700 Ludwigshafen(DE)**

(72) Erfinder: **Rendenbach-Müller, Beatrice, Dr.**
**Kapellenstrasse 8**
**W-6701 Waldsee(DE)**
Erfinder: **Weifenbach, Harald, Dr.**
**Londoner Ring 71**
**W-6700 Ludwigshafen(DE)**
Erfinder: **Teschendorf, Hans-Jürgen, Dr.**
**Georg-Nuss-Strasse 5**
**W-6724 Dudenhofen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

## Beschreibung

Gegenstand der Erfindung sind neue Arylalkoxycumarine der allgemeinen Formel I, die wertvolle therapeutische, insbesondere zur Behandlung zentralnervöser Erkrankungen geeignete Eigenschaften aufweisen, sowie Methoden zu ihrer Herstellung.

Herstellung und gewisse (mikrobizide und UV-absorbierende, jedoch nicht pharmakologische) Eigenschaften von 7-Benzoxycumarin und seinen in 3- oder 4-Stellung methylierten oder phenylierten Derivaten sind z.B. aus folgenden Literaturstellen bekannt:
J. Chem. Soc., Chem. Commun. (16), 1264-6 (CA 106:119499s);
Nippon Kagaku Kaishi (1), 96-9 (CA 82:149030k);
Phytochemistry 10 (12), 2965-70;
Experientia 26 (11), 1281-3;
J. Chem. Ecol. 13 (4), 917-24;
Chem. Pharm. Bull. 28 (12), 3662-4;
Indian J. Chem., Sect. B, 25B (12), 1253-4;
J. Indian Chem. Soc., 63 (4), 442-3;
Indian J. Chem., Sect. B, 25B (8), 862-5;
Curr. Sci. 53 (7), 369-71;
US 3 712 947; US 3 625 976; US 3 351 482.

Ferner ist für 7-Pentafluorphenylmethoxy-4-methylcumarin aus J. Agric. Food Chem. 34 (2), 185-8 eine fungizide Aktivität bekannt.

Der Erfindung lag die Aufgabe zugrunde, neue Therapeutika zur Behandlung zentral nervöser Erkrankungen zu entwickeln.

Die Lösung dieser Aufgabe besteht in den Alkoxycumarinen der allgemeinen Formel I nach Anspruch 1, in dem Verfahren zu deren Herstellung nach Anspruch 2 und den therapeutischen Mitteln nach den Ansprüchen 3 und 4.

In der allgemeinen Formel I

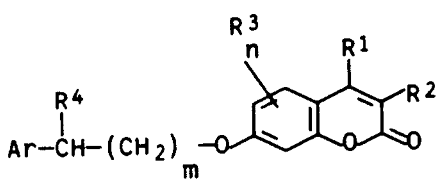

I,

bedeuten $R^1$ und $R^2$, die gleich oder verschieden sein können, Wasserstoff, Niederalkyl, wobei $R^1$ und $R^2$ eine gemeinsame Kette von 3 bis 5 Kohlenstoffatomen bilden können, Phenyl oder Halogen;

- $R^3$ Niederalkyl, Halogen;
- n eine ganze Zahl von 0 bis 3;
- m eine ganze Zahl von 0 bis 4;
- $R^4$ Wasserstoff oder Niederalkyl;
- Ar einen gegebenenfalls ein- bis dreifach durch Halogen, $C_1$-$C_6$-Alkyl oder $C_1$-$C_6$-Alkoxy oder einfach durch Nitro, Cyano oder Trifluormethyl oder eine Kombination dieser Substituenten substituierten Phenyl- oder einen Naphthylring, mit der Maßgabe, daß $m \neq 0$, wenn Ar = unsubstituiertes Phenyl.

Unter "Niederalkyl" ist hier $C_1$- bis $C_5$-Alkyl zu verstehen, unter "Halogen" Fluor, Brom und vor allem Chlor.

Die Verbindungen der allgemeinen Formel I lassen sich beispielsweise herstellen, indem man ein Hydroxycumarin der Formel II

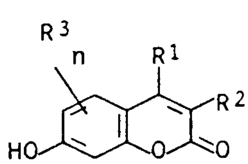

II,

in der $R^1$, $R^2$, $R^3$ und n die oben angegebenen Bedeutungen haben, in an sich bekannter Weise mit einer Verbindung der Formel III

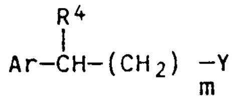

III,

in der $R^4$, m und Aryl wie eingangs definiert sind und Y für eine nucleofuge Abgangsgruppe wie Chlor, Brom oder $R^6SO_2O$ steht, umsetzt. $R^6$ bedeutet hierin Niederalkyl oder gegebenenfalls durch Niederalkyl oder Halogen substituiertes Phenyl. Die Umsetzung kann, wie beispielsweise in Houben-Weyl, Georg Thieme-Verlag, Stuttgart 1965, Bd. 6/3, S. 54 ff. beschrieben, durch Erhitzen der beiden Komponenten, vorzugsweise in Anwesenheit eines inerten Lösungsmittels wie Benzol, Toluol, Methylenchlorid, Aceton, einem niederen Alkohol, Dimethylformamid oder Wasser, auf Temperaturen zwischen Raumtemperatur und dem Siedepunkt des verwendeten Lösungsmittels gewünschtenfalls unter Zusatz katalytischer Mengen Natriumiodid durchgeführt werden. Die freiwerdende Säure wird im allgemeinen durch Zusatz von Basen wie Alkali- oder Erdalkalihydroxiden oder -carbonaten oder Aminen wie Pyridin oder Triethylamin abgefangen. Anstelle der Hydroxycumarine der Formel II können deren Alkalimetallsalze mit den Verbindungen der Formel III, vorzugsweise unter wasserfreien Bedingungen in aprotischen Lösungsmitteln wie Ether, Tetrahydrofuran, Dimethylformamid, Dimethoxyethan oder Dimethylsulfoxid, umgesetzt werden. Als Basen können in diesen Fällen Alkalimetallhydride oder -Alkoholate eingesetzt werden. Die Isolierung und Reinigung der Produkte erfolgt nach an sich bekannten Methoden, beispielsweise durch Umkristallisieren aus einem Lösungsmittel, durch Extraktion oder durch Säulenchromatographie.

Die Hydroxycumarine der allgemeinen Formel II können nach bekannten Methoden, wie sie zum Beispiel in Elderfield R.C., Heterocyclic Compounds, John Wiley-Verlag, New York 1951, Bd. 2, S. 174 f. beschrieben sind, hergestellt werden, beispielsweise durch Kondensation von Dihydroxybenzolen der Formel IV

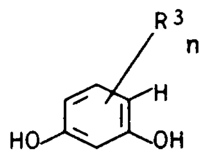

IV,

in der $R^3$ und n die oben angegenenen Bedeutungen haben, mit $\beta$-Ketocarbonsäuren der Formel V

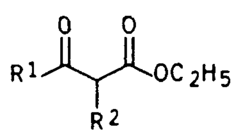

V,

in der $R^1$ und $R^2$ die angegebenen Bedeutungen besitzen, in Anwesenheit eines Kondensationsmittels wie Schwefelsäure, Phoshorpentoxid oder Aluminiumchlorid.

Die Arylverbindungen der allgemeinen Formel III sind bekannt und zum größten Teil kommerziell erhältlich.

Die Verbindungen der Formel I haben Monoaminoxidase(MAO)-inhibierende Aktivität. Aufgrund dieser Aktivität können die Verbindungen der Formel I zur Behandlung von zentralnervösen - insbesondere von neurodegenerativen - Erkrankungen und Parkinsonismus verwendet werden.

Die MAO hemmende Aktivität der erfindungsgemäßen Verbindungen kann unter Verwendung von Standardmethoden bestimmt werden. So erfolgte die Bestimmung der Monoaminooxidasen A und B in verdünntem Rattenhirnhomogenat, dem 1. unterschiedliche Konzentrationen der zu prüfenden Testsubstanzen und 2. $^{14}C$-Phenylethylamin bzw. $^{14}C$-Tryptamin in einer Konzentration von 0,4 µmol/l zugesetzt wurden. Dieser Ansatz wurde 20 min. bei 37 °C inkubiert. Die Reaktion wurde dann durch 0,1 normale HCl gestoppt und die Reaktionsprodukte nach Extraktion im Toluol-Szintillator (PPO + POPOP in Toluol) bestimmt. Der Blindwert wurde in analogen Ansätzen mit einer Inkubationszeit von t = 0 min bestimmt.

Aus den bei den verschiedenen Inhibitor-Konzentrationen gegen die Kontrolle ermittelten Hemmwerten wurde durch lineare Regression nach logit-log-Transformation die mittlere Hemmkonzentration (IC50) berechnet.

Die so ermittelte Aktivität einiger erfindungsgemäßer Verbindungen ist aus der folgenden Tabelle ersichtlich:

| Beispiel | IC50 [µmol/l] | | MAO A / MAO B |
|---|---|---|---|
| | MAO A | MAO B | |
| 1 | >10 | 0,007 | >1400 |
| 4 | >10 | 0,011 | > 900 |
| 6 | >10 | 0,032 | > 300 |
| 7 | > 0,54 | 0,0011 | 490 |
| 8 | 0,39 | 0,00089 | 430 |
| 9 | 0,56 | 0,00087 | 640 |
| 10 | >10 | 0,0037 | >2700 |
| 12 | 2,1 | 0,00059 | 3500 |
| 19 | >10 | 0,0018 | >5500 |
| 20 | 0,3 | 0,0013 | 230 |
| 23 | >10 | 0,013 | > 770 |
| 26 | >10 | 0,0018 | >5600 |
| 27 | >10 | 0,0047 | >2100 |
| 28 | >10 | 0,0028 | >3600 |
| 33 | >10 | 0,0043 | >2400 |
| 34 | 1,5 | 0,0035 | 430 |
| 35 | 1,2 | 0,0042 | 280 |
| 38 | >10 | 0,031 | > 320 |
| Deprenyl | 2,0 | 0,0078 | 256 |
| Ro 19-6327 | >10 | 0,022 | > 450 |

Die erfindungsgemäßen Verbindungen können in üblicher Weise oral oder parenteral (subkutan, intravenös, intramuskulär, intraperitoneal) verabfolgt werden.

Die Dosierung hängt vom Alter, Zustand und Gewicht des Patienten sowie von der Applikationsart ab. In der Regel beträgt die tägliche Wirkstoffdosis zwischen etwa 10 und 500 mg pro Patient und Tag bei oraler und zwischen etwa 1 und 50 mg pro Patient und Tag bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Konzentration von 1 bis 99 Gew.-%.

Beispiel 1

3,4-Dimethyl-7-(4-isopropylphenyl)-methoxycumarin

5,7 g 7-Hydroxy-3,4-dimethylcumarin in 25 ml DMF wurden bei Raumtemperatur zu einer Suspension von 1,05 g NaH (80 %) in 15 ml DMF zugetropft. Nach 45 min wurde mit 5,05 g 4-Isopropylbenzylchlorid gelöst in 20 ml DMF versetzt und über Nacht bei RT gerührt. Die Reaktionsmischung wurde mit Eiswasser hydrolysiert, der ausgefallene Feststoff abgesaugt und aus Methanol umkristallisiert.

Ausbeute: 4,3 g (45 %): Fp. 96°C

| $C_{21}H_{22}$ (322) | | | |
|---|---|---|---|
| Ber. | 78,23 C | 6,88 H | 14,99 O |
| Gef. | 78,0 C | 7,0 H | 14,8 O |

Beispiel 2

7-(4-Bromphenyl)-methoxy-3,4-dimethylcumarin

Ein Gemisch aus 5,0 g 7-Hydroxy-3,4-dimethylcumarin, 5,4 g 4-Brombenzylchlorid, 5,4 g $K_2CO_3$ und 100 ml Aceton wurde 4 Tage bei Raumtemperatur gerührt, eingeengt und der Rückstand in $H_2O$/Methylenchlorid verteilt. Nach Abtrennen der organischen Phase wurde noch zweimal mit Methylenchlorid extrahiert, die vereinigten organischen Phasen eingeengt und der Rückstand aus Methanol umkristallisiert.

Ausbeute: 5,9 g (62 %) ; Fp. 153 ° C

| $C_{18}H_{15}BrO_3$ (359) | | | | |
|---|---|---|---|---|
| Ber. | 60,18 C | 4,21 H | 22,24 Br | 13,36 O |
| Gef. | 60,1 C | 4,3 H | 22,2 Br | 13,5 O |

Analog Beispiel 1 wurden hergestellt:

Beispiel 3

6-Chlor-3,4-dimethyl-7-(4-isopropylphenyl)-methoxycumarin

Durchführung und Aufarbeitung erfolgten analog Beispiel 2.

Ausbeute: 47 %; Fp. 168 ° C (Essigsäureethylester)

| $C_{21}H_{21}ClO_3$ (357) | | | | |
|---|---|---|---|---|
| Ber. | 70,68 C | 5,93 H | 9,94 Cl | 13,45 O |
| Gef. | 70,3 C | 6,0 H | 9,8 Cl | 13,5 O |

Beispiel 4

3,4-Dimethyl-7-(2-naphthyl)-methoxycumarin

Ausbeute: 62 %; Fp. 161-164 ° C (Methanol)

| $C_{22}H_{18}O_3$ (330) | | | |
|---|---|---|---|
| Ber. | 79,50 C | 6,06 H | 14,44 O |
| Gef. | 79,4 C | 5,8 H | 14,1 O |

Beispiel 5

3,4-Dimethyl-7-(1-naphthyl)-methoxycumarin

Ausbeute: 45 %; Fp. 186-189°C (Methanol)

| $C_{22}H_{18}O_3$ (330) | | | |
|---|---|---|---|
| Ber.<br>Gef. | 79,5 C<br>79,3 C | 6,06 H<br>5,7 H | 14,44 O<br>14,5 O |

Beispiel 6

7-(4-t-Butylphenyl)-methoxy-3,4-dimethylcumarin

Ausbeute: 47 %; Fp. 112-113°C (Methanol)

| $C_{22}H_{24}O_3$ (336) | | | |
|---|---|---|---|
| Ber.<br>Gef. | 78,54 C<br>78,4 C | 7,19 H<br>7,5 H | 14,27 O<br>13,9 O |

Beispiel 7

3,4-Dimethyl-7-(2-methylphenyl)-methoxycumarin

Ausbeute: 81 %; Fp. 145°C (Methanol)

| $C_{19}H_{18}O_3$ (294) | | | |
|---|---|---|---|
| Ber.<br>Gef. | 77,53 C<br>77,6 C | 6,16 H<br>6,3 H | 16,31 O<br>16,3 O |

Beispiel 8

3,4-Dimethyl-7-(3-methylphenyl)-methoxycumarin

Ausbeute: 85 %; Fp. 114°C (Methanol)

| $C_{19}H_{18}O_3$ (294) | | | |
|---|---|---|---|
| Ber.<br>Gef. | 77,53 C<br>77,8 C | 6,16 H<br>6,1 H | 16,31 O<br>16,1 O |

Beispiel 9

3,4-Dimethyl-7-(4-methylphenyl)-methoxycumarin

Die Reaktionsmischung wurde 3 h bei 60°C und über Nacht bei RT gerührt. Ansatz und Aufarbeitung erfolgten wie unter Beispiel 1 beschrieben.

Ausbeute: 76 %, Fp.: 123 °C (Methanol)

| $C_{19}H_{18}O_3$ (294) | | | |
|---|---|---|---|
| Ber. | 77,53 C | 6,16 H | 16,31 O |
| Gef. | 77,6 C | 6,3 H | 16,2 O |

Beispiel 10

3,4-Dimethyl-7-(2,5-dimethylphenyl)-methoxycumarin

Die Reaktionsdurchführung erfolgte wie unter Beispiel 9 beschrieben.
Ausbeute: 82 %, Fp. 173-175°C (Essigsäureethylester)

| $C_{20}H_{20}O_3$ (308) | | | |
|---|---|---|---|
| Ber. | 77,90 C | 6,54 H | 15,56 O |
| Gef. | 77,7 C | 6,6 H | 15,2 O |

Beispiel 11

3,4-Dimethyl-7-(2,4,6-trimethylphenyl)-methoxycumarin

Die Reaktionsmischung wurde 3 h bei 60°C und über Nacht bei RT gerührt. Ansatz und Aufarbeitung erfolgten wie unter Beispiel 1 beschrieben.
Ausbeute: 50 %, Fp. 175-181°C (Essigsäureethylester)

| $C_{21}H_{22}O_3$ (322) | | | |
|---|---|---|---|
| Ber. | 78,23 C | 6,88 H | 14,89 O |
| Gef. | 78,1 C | 6,9 H | 14,7 O |

Beispiel 12

7-(4-Methoxyphenyl)-methoxy-3,4-dimethylcumarin

Die Reaktionsdurchführung erfolgte wie unter Beispiel 9 beschrieben.
Ausbeute: 66 %; Fp. 130-132°C (Essigsäureethylester)

| $C_{19}H_{18}O_4$ (310) | | | |
|---|---|---|---|
| Ber. | 73,53 C | 5,85 H | 20,62 O |
| Gef. | 73,3 C | 5,9 H | 20,3 O |

Beispiel 13

3,4-Dimethyl-7-(4-nitrophenyl)-methoycumarin

Ansatz und Durchführung erfolgten wie unter Beispiel 1 beschrieben. Der nach der Hydrolyse ausgefallene Feststoff wurde abgesaugt, nacheinander mit je 500 ml Heptan und Aceton ausgekocht und der Rückstand im Vakuum getrocknet.
Ausbeute: 25 % Fp. 298-299°C

| $C_{18}H_{25}NO_5$ (335) | | | | |
|---|---|---|---|---|
| Ber.<br>Gerf. | 66,46 C<br>66,2 C | 4,65 H<br>4,6 H | 4,51 N<br>4,5 N | 24,59 O<br>24,4 O |

Beispiel 14

7-(4-Fluorphenyl)-methoxy-3,4-dimethylcumarin

Ausbeute: 44 %; Fp. 142°C (Essigsäureethylester)

| $C_{18}H_{15}FO_3$ (298) | | | | |
|---|---|---|---|---|
| Ber.<br>Gef. | 72,47 C<br>72,3 C | 5,07 H<br>5,2 H | 6,37 F<br>6,5 F | 16,09 O<br>16,0 O |

Beispiel 15

7-(4-Chlorphenyl)-methoxy-3,4-dimethylcumarin

Ausbeute: 30 %; Fp. 148°C (Essigsäureethylester)

| $C_{18}H_{15}ClO_3$ (315) | | | | |
|---|---|---|---|---|
| Ber.<br>Gef. | 68,69 C<br>68,2 C | 4,8 H<br>4,9 H | 11,26 Cl<br>11,2 Cl | 15,25 O<br>15,5 O |

Beispiel 16

7-(4-Cyanophenyl)-methoxy-3,4-dimethylcumarin

Ausbeute: 67 %; Fp. 175-176°C (Methanol)

| $C_{19}H_{15}NO_3$ (305) | | | | |
|---|---|---|---|---|
| Ber.<br>Gef. | 74,75 C<br>74,3 C | 4,96 H<br>4,9 H | 4,58 N<br>4,4 N | 15,7 O<br>16,2 O |

Beispiel 17

7-(3-Chlorphenyl)-methoxy-3,4-dimethylcumarin

Ausbeute: 74 %; Fp. 141 °C (Essigsäureethylester)

| $C_{18}H_{15}ClO_3$ (315) | | | | |
|---|---|---|---|---|
| Ber.<br>Gef. | 68,69 C<br>68,5 C | 4,8 H<br>4,9 H | 11,26 Cl<br>11,1 Cl | 15,25 O<br>15,2 O |

Beispiel 18

7-(3-Cyanophenyl)-methoxy-3,4-dimethylcumarin

Ausbeute: 48 %; Fp. 178-183°C (Methanol)

| $C_{19}H_{15}NO_3$ (305) | | | | |
|---|---|---|---|---|
| Ber. | 74,75 C | 4,96 H | 4,58 N | 15,7 O |
| Gef. | 74,5 C | 5,1 H | 4,7 N | 15,5 O |

Beispiel 19

7-(4-Trifluormethylphenyl)-methoxy-3,4-dimethylcumarin

Ausbeute 62 %; Fp. 157-160°C (Methanol)

| $C_{19}H_{15}F_3O$ (348) | | | | |
|---|---|---|---|---|
| Ber. | 65,52 C | 4,34 H | 16,36 F | 13,78 O |
| Gef. | 65,5 C | 4,4 H | 16,8 F | 13,3 O |

Beispiel 20

7-(3-Trifluormethylphenyl)-methoxy-3,4-dimethylcumarin

Ausbeute: 56 %; Fp. 136-138°C (Methanol)

| $C_{19}H_{15}F_3O$ (348) | | | | |
|---|---|---|---|---|
| Ber. | 65,52 C | 4,34 H | 16,36 F | 13,78 O |
| Gef. | 65,4 C | 4,4 H | 16,9 F | 13,3 O |

Beispiel 21

3,4-Dimethyl-7-(2-phenyl)-ethoxycumarin

Ansatz und Durchführung erfolgten wie unter Beispiel 1 beschrieben. Der nach Hydrolyse und Abdekantieren des Lösungsmittels verbleibende ölige Rückstand wurde in Methylenchlorid aufgenommen, mit 2N NaOH-Lösung und mit Wasser gewaschen und über $Na_2SO_4$ getrocknet. Der nach Filtration und Entfernen des Lösungsmittels verbleibende Feststoff wurde aus wenig Methanol umkristallisiert.

Ausbeute: 20 %; Fp. 116°C

| $C_{19}H_{18}O_3$ (294) | | | |
|---|---|---|---|
| Ber. | 77,53 C | 6,16 H | 16,31 O |
| Gef. | 77,4 C | 6,3 H | 16,6 O |

Beispiel 22

3,4-Dimethyl-7-[1-(4-isopropylphenyl)]-ethoxycumarin

Ansatz und Durchführung erfolgten wie unter Beispiel 1 beschrieben. Nach der Hydrolyse wurde mit Methyl-t-butylether extrahiert, die organische Phase mit $H_2O$ gewaschen, getrocknet und der nach dem Einengen verbleibende Feststoff aus Methanol umkristallisiert.

Ausbeute: 20 %; Fp. 152-153°C

Beispiel 23

6-Ethyl-3,4-dimethyl-7-(2-phenyl )-ethoxycumarin

Ausbeute: 35 %; Fp. 123°C (Methanol)

| $C_{21}H_{22}O_3$ (322) | | | |
|---|---|---|---|
| Ber. | 78,23 C | 6,88 H | 14,99 O |
| Gef. | 78,5 C | 7,2 H | 14,4 O |

Beispiel 24

3,4,8-Trimethyl-7-(4-isopropylphenyl)-methoxycumarin

Ausbeute: 49 %, Fp. 172 °C (Methanol)

| $C_{22}H_{24}O_3$ (336) | | | |
|---|---|---|---|
| Ber. | 78,54 C | 7,19 H | 14,27 O |
| Gef. | 78,4 C | 7,4 H | 14,2 O |

Beispiel 25

6-Ethyl-3,4-dimethyl-7-(4-isopropylphenyl)-methoxycumarin Ausbeute: 65 %; Fp. 129°C (Methanol)

| $C_{23}H_{26}O_3$ (350) | | | |
|---|---|---|---|
| Ber. | 78,85 C | 7,42 H | 13,71 O |
| Gef. | 78,6 C | 7,6 H | 13,6 O |

Beispiel 26

7-(4-Isopropylphenyl)-methoxycumarin

Ausbeute: 46 %; Fp. 127°C (Methanol)

| $C_{19}H_{18}O_3$ (294) | | | |
|---|---|---|---|
| Ber. | 77,53 C | 6,16 H | 16,31 O |
| Gef. | 77,3 C | 6,2 H | 16,3 O |

Beispiel 27

4-methyl-7-(4-isopropylphenyl)-methoxycumarin

Ausbeute: 46 %; Fp. 156°C (Methanol)

| $C_{20}H_{20}O_3$ (308) | | | |
|---|---|---|---|
| Ber. | 77,90 C | 6,54 H | 15,56 O |
| Gef. | 77,9 C | 6,7 H | 15,6 O |

Beispiel 28

3-methyl-7-(4-isopropylphenyl)-methoxycumarin

Ausbeute: 42 %; Fp. 129°C (Methanol)

| $C_{20}H_{20}O_3$ (308) | | | |
|---|---|---|---|
| Ber. | 77,90 C | 6,54 H | 15,56 O |
| Gef. | 77,4 C | 6,6 H | 15,4 O |

Beispiel 29

3-Ethyl-4-methyl-7-(4-isopropylphenyl)-methoxycumarin

Ausbeute: 64 %; Fp. 115°C (Methanol)

| $C_{20}H_{24}O_3$ (336) | | | |
|---|---|---|---|
| Ber. | 78,54 C | 7,19 H | 14,27 O |
| Gef. | 78,6 C | 7,3 H | 14,3 O |

Beispiel 30

4-Ethyl-3-methyl-7-(4-isopropylphenyl)-methoxycumarin

Ausbeute: %; Fp. 83 °C (Methanol)

Beispiel 31

3,4-Tetramethylen-7-(4-isopropylphenyl)-methoxycumarin

Ausbeute: 39 %; Fp. 129°C (Methanol)

| $C_{23}H_{24}O_3$ (348) | | | |
|---|---|---|---|
| Ber. | 79,28 C | 6,94 H | 13,77 O |
| Gef. | 79,5 C | 7,0 H | 13,7 O |

Beispiel 32

4-Phenyl-7-(4-isopropylphenyl)-methoxycumarin

Ansatz und Durchführung erfolgten wie unter Beispiel 1 beschrieben. Nach der Hydrolyse wurde mit Methyl-t-butylether extrahiert und die organische Phase getrocknet und eingeengt.
Ausbeute: 48 %; Fp. 85,5 °C (Methanol)

Beispiel 33

3-Chlor-4-methyl-7-(4-isopropylphenyl)-methoxycumarin

Ausbeute: 16 %; Fp. 124°C (Methanol)

| $C_{20}H_{19}ClO_3$ (343) | | | |
|---|---|---|---|
| Ber.<br>Gef. | 70,07 C<br>70,5 C | 5,54 H<br>5,8 H | 14,01 O<br>13,7 O |

Beispiel 34

7-(3-Phenyl)-propoxycumarin

Die Herstellung erfolgte wie unter Beispiel 21 beschrieben.
Ausbeute: 43 %, Fp. 124-126 °C (Methanol)

Beispiel 35

3,4-Dimethyl-7-(3-phenyl)-propoxycumarin

Die Durchführung erfolgte wie unter Beispiel 21 beschrieben.
Ausbeute: 51 %; Fp. 104 ° C (Methanol)

| $C_{20}H_{20}O_3$ (308) | | | |
|---|---|---|---|
| Ber.<br>Gef. | 77,90 C<br>77,8 C | 6,54 H<br>6,6 H | 15,56 O<br>15,4 O |

Beispiel 36

7-(5-phenyl)-pentoxycumarin

Die Durchführung erfolgte wie unter Beispiel 21 beschrieben.
Ausbeute: 37 %; Fp. 103 ° C (Methanol).

Beispiel 37

3,4-Dimethyl-7-(5-phenyl)-pentoxycumarin

Die Durchführung erfolgte wie unter Beispiel 21 beschrieben.
Ausbeute: 32 %; Fp. 101 ° C (Methanol)

Beispiele für galenische Applikationsformen:

A) Auf einer Tablettenpresse werden in üblicher Weise Tabletten folgender Zusammensetzung gepreßt:
40 mg Substanz des Beispiels 1
120 mg Maisstärke
13,5 mg Gelatine
45 mg Milchzucker
2,25 mg Aerosil® (chemisch reine Kieselsäure in submikroskopisch feiner Verteilung)
6,75 mg Kartoffelstärke (als 6%iger Kleister)
B) 20 mg Substanz des Beispiels 3
60 mg Kernmasse
60 mg Verzuckerungsmasse

Die Kernmasse besteht aus 9 Teilen Maisstärke, 3 Teilen Milchzucker und 1 Teil Luviskol® VA 64 (Vinypyrrolidon-Vinylacetat-Misch-polymerisat 60 : 40, vgl. Pharm.Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

## Patentansprüche

**1.** Arylalkoxycumarine der allgemeinen Formel I

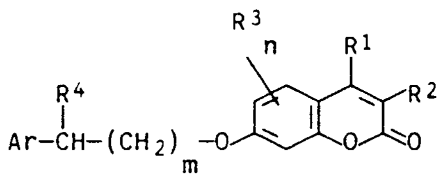

I,

in der $R^1$ und $R^2$ unabhängig voneinander Wasserstoff, Niederalkyl, Phenyl, Halogen oder beide gemeinsam eine Alkylenbrücke mit 3 bis 5 Kohlenstoffatomen darstellen und
$R^3$ Niederalkyl oder Halogen;
n eine ganze Zahl von 0 bis 3;
m eine ganze Zahl von 0 bis 4;
$R^4$ Wasserstoff oder Niederalkyl;
Ar einen gegebenenfalls ein- bis dreifach durch Halogen, $C_1$-$C_6$-Alkyl- oder $C_1$-$C_6$-Alkoxy oder einfach durch Nitro, Cyano oder Trifluormethyl substituierten Phenyl- oder einen Naphthylrest bedeuten, mit der Maßgabe, daß m nicht gleich 0 ist, wenn Ar einen unsubstituierten Phenylrest darstellt.

**2.** Verfahren zur Herstellung der Verbindungen der Formel I nach Anspruch 1, dadurch gekennzeichnet, daß ein Hydroxycumarin der Formel II

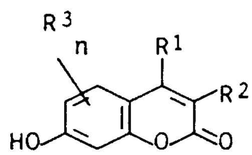

II

mit einer Verbindung der Formel III

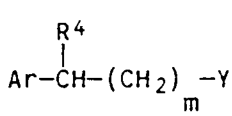

III,

wobei $R^1$ bis $R^4$, Ar, n und m die im Anspruch 1 angegebenen Bedeutungen besitzen und Y eine

nukleofuge Abgangsgruppe darstellt, in an sich bekannter Weise miteinander umsetzt und das Reaktionsprodukt der Formel I nach üblichen Methoden isoliert.

**3.** Orales therapeutisches Mittel, das als Wirkstoff pro Dosis 10 bis 500 mg einer Verbindung der allgemeinen Formel I nach Anspruch 1 neben üblichen galenischen Hilfsmitteln enthält.

**4.** Parenterales therapeutisches Mittel, das als Wirkstoff pro Dosis 1 bis 50 mg einer Verbindung der allgemeinen Formel I nach Anspruch 1 neben üblichen galenischen Hilfsmitteln enthält.

**5.** Mittel zur Behandlung von Erkrankungen des zentralen Nervensystems, das als Wirkstoff eine Verbindung der allgemeinen Formel I nach Anspruch 1 neben üblichen galenischen Hilfsmitteln enthält.

## Claims

**1.** An arylalkoxycoumarin of the formula I

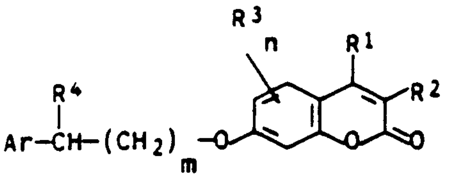

I

where $R^1$ and $R^2$ independently of one another are each hydrogen, lower alkyl, phenyl or halogen, or the two radicals together form an alkylene bridge of 3 to 5 carbon atoms, $R^3$ is lower alkyl or halogen, n is an integer of from 0 to 3, m is an integer of from 0 to 4, $R^4$ is hydrogen or lower alkyl and Ar is a phenyl ring which is unsubstituted or monosubstituted to trisubstituted by halogen, $C_1$-$C_6$-alkyl or $C_1$-$C_6$-alkoxy or monosubstituted by nitro, cyano or trifluoromethyl or is a naphthyl ring, with the proviso that m is not 0 when Ar is unsubstituted phenyl.

**2.** A process for the preparation of a compound of the formula I as claimed in claim 1, wherein a hydroxycoumarin of the formula II

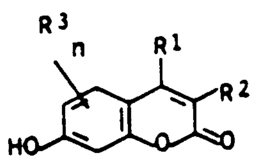

II

is reacted with a compound of the formula III

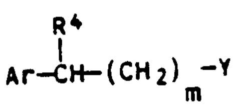

III

where $R^1$ to $R^4$, Ar, n and m have the meanings stated in claim 1 and Y is a nucleofugic leaving group, in a conventional manner, and the reaction product of the formula I is isolated by a usual method.

**3.** An oral therapeutic agent which contains, as the active compound, from 10 to 500 g, per dose, of a compound of the formula I as claimed in claim 1, in addition to conventional pharmaceutical auxiliaries.

**4.** A parenteral therapeutic agent which contains, as the active compound, from 1 to 50 mg, per dose, of a compound of the formula I as claimed in claim 1, in addition to conventional pharmaceutical auxiliaries.

**5.** An agent for treating disorders of the central nervous system, which contains, as the active compound, a compound of the formula I as claimed in claim 1, in addition to conventional pharmaceutical

auxiliaries.

**Revendications**

1. Arylalcoxycoumarines de formule générale I

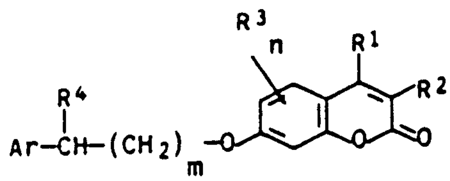

I,

dans laquelle $R^1$ et $R^2$, indépendamment l'un de l'autre, représentent hydrogène, alkyle inférieur, phényle, halogène ou les deux, ensemble, un pont alkylène de 3 à 5 atomes de carbone,
$R^3$ = alkyle inférieur ou halogène;
n = un nombre entier de 0 à 3;
m = un nombre entier de 0 à 4;
$R^4$ = hydrogène ou alkyle inférieur;
Ar = un reste phényle éventuellement substitué une à trois fois par halogène, alkyle en $C_{1-6}$ ou alcoxy en $C_{1-6}$, ou une fois par nitro, cyano ou trifluorométhyle, ou un reste naphtyle, étant entendu que m ne représente pas O si Ar est un reste phényle non substitué.

2. Procédé de préparation des composés de formule I selon la revendication 1, caractérisé par le fait que l'on met à réagir, de manière connue en soi, une hydroxycoumarine de formule II

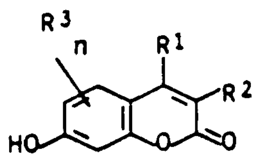

II

avec un composé de formule III

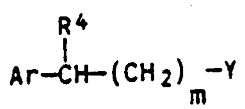

III,

$R^1$ à $R^4$, Ar, n et m ayant les significations données dans la revendication 1 et Y représentant un groupe nucléofuge éliminable, et on isole le produit de réaction de formule I selon des méthodes usuelles.

3. Agent thérapeutique buccal, qui, outre des agents auxiliaires galéniques usuels, contient, comme principe actif, 10 à 500 mg/dose d'un composé de formule générale I, selon la revendication 1.

4. Agent thérapeutique parentéral, qui , outre des agents auxiliaires galéniques usuels, contient, comme principe actif, 1 à 50 mg/dose d'un composé de formule générale I, selon la revendication 1.

5. Médicament pour le traitement de maladies du système nerveux central, qui, outre des agents auxiliaires galéniques usuels, contient, comme principe actif, un composé de formule générale I, selon la revendication 1.